# EUROPEAN PATENT APPLICATION

(11) **EP 3 299 795 A1**
(43) Date of publication of application: **28.03.2018**
(21) Application number: 17191813.9
(22) Date of filing: 19.09.2017
(51) Int. Cl.: G01N 5/04

(54) **DYNAMIC MOISTURE ABSORPTION-DESORPTION PROPERTY EVALUATION APPARATUS, METHOD FOR EVALUATING DYNAMIC MOISTURE ABSORPTION-DESORPTION PROPERTY , AND DYNAMIC MOISTURE ABSORPTION-DESORPTION PROPERTY EVALUATION PROGRAM**

(30) Priority: 23.09.2016 JP 2016186127
(71) Applicant: Nitto Denko Corporation, Ibaraki-shi, Osaka 567-8680 (JP)
(72) Inventor: Kishi, Atsushi, Osaka, 567-8680 (JP); Ishihara, Yasutaka, Osaka, 567-8680 (JP); Mamiya, Satoru, Osaka, 567-8680 (JP)
(74) Representative: Hoffmann Eitle

(57) **Abstract**

A water vapor concentration in a test gas in a measurement chamber in which a sample is disposed is adjusted, and a moisture amount of the sample in the measurement chamber is measured by a measurement unit. When the water vapor concentration in the test gas in the measurement chamber is changed, a shift B in change of the moisture amount of the sample measured by the measurement unit, from change of the water vapor concentration in the test gas, is calculated. The calculated shift B in the change of the moisture amount of the sample corresponds to an adsorption-desorption rate of the sample. Thus, it is possible to analyze not only the moisture amount of the sample but also the adsorption-desorption rate of the sample, so that characteristics of the sample can be widely analyzed.

## Description

### TECHNICAL FIELD

The present invention relates to a dynamic moisture absorption-desorption property evaluation apparatus, a method for evaluating a dynamic moisture absorption-desorption property, and a dynamic moisture absorption-desorption property evaluation program, for evaluating a dynamic moisture absorption-desorption property of a sample in a measurement chamber.

### BACKGROUND

Conventionally, as shown in paragraph [0137] and the like of Japanese Unexamined Patent Publication No. 2015-52765, a moisture amount measuring apparatus (e.g., a moisture adsorption-desorption measuring apparatus "IGA-Sorp" manufactured by Hiden Analytical Ltd.) for measuring moisture amount of a sample has been used. Using this type of apparatus enables measurement of the amount of moisture contained in various samples, such as a single film and a multilayer film having a multilayer laminated structure.

For example, a polarizing plate is a multilayer film in which a base film, a polarizer, an adhesive layer, and the like are laminated. A polarizing plate potentially has low durability in a wet heat test, and the moisture permeability and moisture amount in each layer thereof are very important. Each layer constituting a polarizing plate has its own ease of moisture accumulation, and when each layer is individually measured using a moisture amount measuring apparatus, the moisture amount of each layer can be calculated. It has been thought that durability of a polarizing plate in which these layers are laminated can be estimated in design based on the moisture permeability and moisture amount of each layer.

However, in a process of studying durability of a polarizing plate for wet heat, the inventors of the present invention have found that ease of moisture accumulation in each layer constituting the polarizing plate is different between in a single layer state and in a laminated state. It is considered that this is caused by receiving interaction such as internal pressure due to difference in linear expansion of each of the laminated layers and existence of internal stress caused through a stretching process.

In a conventional moisture amount measuring apparatus, it is impossible to analyze the moisture amount of each layer in a multilayer film, such as a polarizing plate, in a state where each layer is laminated. Therefore, this does not enable analysis of characteristics (moisture permeability and moisture amount) of each layer of a multilayer film used while each layer is laminated in a state where the multilayer film is actually used. In addition, the conventional moisture amount measuring apparatus can only measure moisture amount of not only the multilayer film but also a sample, and thus it is difficult to widely analyze a moisture absorption-desorption property of a sample when a water vapor concentration is changed (hereinafter referred to as also "dynamic moisture absorption-desorption property").

Further, such a problem is a new problem that arises not only in the case of analyzing a dynamic moisture absorption-desorption property of a sample when water vapor is used as a specimen, but also in the case of analyzing a dynamic absorption-desorption property of a sample when another specimen gas such as oxygen, carbon dioxide, or the like is used.

The present invention has been made in light of the above-mentioned circumstances, and it is an object of the present invention to provide a dynamic moisture absorption-desorption property evaluation apparatus, a method for evaluating a dynamic moisture absorption-desorption property, and a dynamic moisture absorption-desorption property evaluation program, capable of widely analyzing characteristics of a sample. Another object of the present invention is to provide a dynamic moisture absorption-desorption property evaluation apparatus, a method for evaluating a dynamic moisture absorption-desorption property, and a dynamic moisture absorption-desorption property evaluation program, capable of evaluating characteristics in any layer in a sample.

### SUMMARY OF THE INVENTION

(1) A dynamic moisture absorption-desorption property evaluation apparatus according to the present invention includes a measurement chamber, a test gas flow system, a test gas adjustment system, a measurement unit, a control unit, and an analysis unit. A sample is disposed in the measurement chamber. The test gas flow system allows a test gas to continuously flow into the measurement chamber. The test gas adjustment system adjusts a water vapor concentration in the test gas in the measurement chamber. The measurement unit measures the moisture amount of the sample in the measurement chamber. The control unit causes the test gas adjustment system to change the water vapor concentration in the test gas. The analysis unit analyzes a shift in change of the moisture amount of the sample measured by the measurement unit from change of the water vapor concentration in the test gas, when the water vapor concentration in the test gas is changed by the control unit.

According to the configuration described above, it is possible to analyze characteristics of a sample based on the shift in change of the moisture amount of the sample from change of the water vapor concentration in the test gas, the shift being calculated by changing the water vapor concentration in the test gas. The calculated shift in the change of the moisture amount of the sample corresponds to an adsorption-desorption rate in the sample. Thus, it is possible to analyze not only the moisture amount of the sample but also an adsorption-desorption rate of the sample, so that characteristics of the sample can be widely analyzed.
(2) The control unit may cause the test gas adjustment system to periodically change the water vapor concentration in the test gas. In this case, the analysis unit may perform waveform analysis based on a waveform indicating a periodic change of the water vapor concentration in the test gas, and a waveform indicating a periodic change of the moisture amount of the sample measured by the measurement unit, with respect to the change of the water vapor concentration in the test gas.

According to the configuration described above, the waveform analysis when the water vapor concentration in the test gas is periodically changed enables the characteristics of the sample to be widely analyzed. Specifically, a height of the waveform corresponds to ease of moisture accumulation, and a shift in the waveforms is caused by an adsorption-desorption rate, so that the characteristics of the sample can be analyzed. In addition, changing a frequency of the periodic change of the water vapor concentration in the test gas stepwise or continuously leads to evaluation of detailed characteristics of the sample based on frequency dependence of the water vapor concentration in the test gas.
(3) The control unit may change a frequency of periodic change of the water vapor concentration in the test gas stepwise or continuously. In this case, the analysis unit may perform waveform analysis at each frequency.

According to the configuration described above, when the waveform analysis is performed at each frequency, an internal structure of the sample can be evaluated based on frequency dependence of the water vapor concentration in the test gas.
(4) The test gas adjustment system may adjust the water vapor concentration and temperature in the test gas. In this case, the control unit may cause the test gas adjustment system to change the water vapor concentration and temperature in the test gas. In addition, the analysis unit may calculate a shift in change of the moisture amount of the sample measured by the measurement unit from change of the water vapor concentration in the test gas at each temperature, when temperature of the test gas is changed by the control unit and the water vapor concentration in the test gas at each temperature is changed by the control unit.

According to the configuration described above, when a shift in change of the moisture amount of the sample from change of the water vapor concentration in the test gas at each temperature is calculated by changing temperature of the test gas, characteristics of the sample can be analyzed based on the shift.

The control unit may cause the test gas adjustment system to periodically change temperature of the test gas. In this case, the analyzing unit may perform waveform analysis based on a waveform indicating a periodic change of the temperature of the test gas and a waveform indicating a periodic change of the moisture amount of the sample measured by the measurement unit, with respect to the change in the temperature. Changing a frequency of the periodic change in the temperature stepwise or continuously leads to evaluation of detailed characteristics of the sample based on frequency dependency of the temperature of the test gas.
(5) A dynamic absorption-desorption property evaluation apparatus according to the present invention includes a measurement chamber, a test gas flow system, a test gas adjustment system, a measurement unit, a control unit, and an analysis unit. A sample is disposed in the measurement chamber. The test gas flow system allows a test gas to continuously flow into the measurement chamber. The test gas adjustment system adjusts a concentration of a specimen in the test gas in the measurement chamber. The measurement unit measures the absorption-desorption amount of the specimen in the sample in the measurement chamber. The control unit causes the test gas adjustment system to change a concentration of the specimen in the test gas. The analysis unit analyzes a shift in change of the absorption-desorption amount of the specimen in the sample measured by the measurement unit from change of the concentration of the specimen in the test gas, when the concentration of the specimen in the test gas is changed by the control unit.

According to the configuration described above, it is possible to analyze characteristics of a sample based on the shift in change of the absorption-desorption amount of the specimen in the sample from change of the concentration of the specimen in the test gas, the shift being calculated by changing the concentration of the specimen in the test gas. The calculated shift in the change of the absorption-desorption amount of the specimen in the sample corresponds to an adsorption-desorption rate in the sample. Thus, it is possible to analyze not only the absorption-desorption amount of the sample but also an adsorption-desorption rate of the sample, so that characteristics of the sample can be widely analyzed.
(6) A method for evaluating a dynamic moisture absorption-desorption property according to the present invention includes the steps of: disposing a sample; allowing a test gas to flow; performing control; and performing analysis. In the step of disposing a sample, a sample is disposed in a measurement chamber. In the step of allowing a test gas to flow, a test gas is allowed to continuously flow into the measurement chamber. In the step of performing control, a water vapor concentration in the test gas is changed. In the step of performing analysis, there is performed analysis of a shift in change of the moisture amount of the sample from change of the water vapor concentration in the test gas when the water vapor concentration in the test gas is changed in the step of performing control.
(7) A method for evaluating a dynamic absorption-desorption property according to the present invention includes the steps of: disposing a sample; allowing a test gas to flow; performing control; and performing analysis. In the step of disposing a sample, a sample is disposed in a measurement chamber. In the step of allowing a test gas to flow, a test gas is allowed to continuously flow into the measurement chamber. In the step of performing control, a concentration of a specimen in the test gas is changed. In the step of performing analysis, there is performed analysis of a shift in change of the absorption-desorption amount of a specimen in the sample from change of the concentration of the specimen in the test gas when the concentration of the specimen in the test gas is changed in the step of performing control.
(8) A dynamic moisture absorption-desorption property evaluation program according to the present invention is configured to evaluate a dynamic moisture absorption-desorption property of a sample in a measurement chamber, the program causing a computer to execute the steps of: allowing a test gas to flow; performing control; and performing analysis. In the step of allowing a test gas to flow, a test gas is allowed to continuously flow into the measurement chamber. In the step of performing control, a water vapor concentration in the test gas is changed. In the step of performing analysis, there is performed analysis of a shift in change of the moisture amount of the sample from change of the water vapor concentration in the test gas when the water vapor concentration in the test gas is changed in the step of performing control.
(9) A dynamic absorption-desorption property evaluation program according to the present invention is configured to evaluate a dynamic absorption-desorption property of a sample in a measurement chamber, the program causing a computer to execute the steps of: allowing a test gas to flow; performing control; and performing analysis. In the step of allowing a test gas to flow, a test gas is allowed to continuously flow into the measurement chamber. In the step of performing control, a concentration of a specimen in the test gas is changed. In the step of performing analysis, there is performed analysis of a shift in change of the absorption-desorption amount of a specimen in the sample from change of the concentration of the specimen in the test gas when the concentration of the specimen in the test gas is changed in the step of performing control.

According to the present invention, it is possible to analyze not only the moisture amount of the sample but also the adsorption-desorption rate of the sample, so that characteristics of the sample can be widely analyzed. In addition, according to the present invention, characteristics in any layer in a sample can be evaluated by using waveform analysis.

### BRIEF DESCRIPTION OF THE DRAWINGS

Fig. 1 is a schematic sectional view illustrating a structure of a measurement chamber in a dynamic moisture absorption-desorption property evaluation apparatus according to an embodiment of the present invention;
Fig. 2 is a schematic sectional view illustrating an example of the composition of a sample;
Fig. 3 is a block diagram illustrating an example of a configuration of a dynamic moisture absorption-desorption property evaluation apparatus; and
FIG. 4 is a graph for describing a method for analyzing a sample.

### DETAILED DESCRIPTION OF THE PREFERRED EMBODIMENTS

### 1. Structure of Measurement Chamber

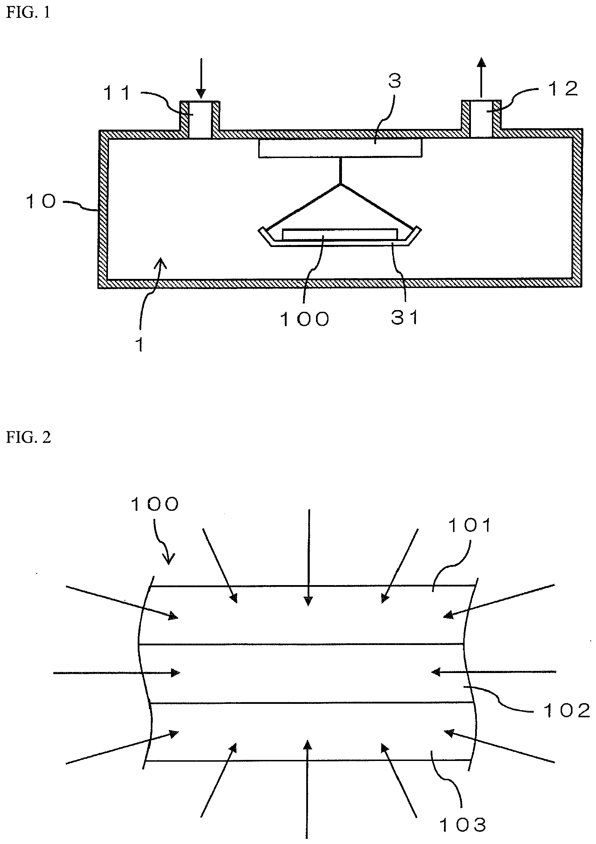

Fig. 1 is a schematic sectional view illustrating a structure of a measurement chamber 1 in a dynamic moisture absorption-desorption property evaluation apparatus according to an embodiment of the present invention. The measurement chamber 1 is formed inside a casing 10. A sample 100 to be measured is disposed in the measurement chamber 1. In the present embodiment, the case where the sample 100 is a film member (film-shaped sample) will be described, but the sample 100 is not limited to the film member.

The casing 10 is provided with an inflow port 11 and an outflow port 12. Gas flows into the measurement chamber 1 from the inflow port 11. The gas flowing into the measurement 1 is a test gas adjusted to a set water vapor concentration (humidity). This allows the measurement chamber 1 to be filled with the test gas, and the test gas exceeding the capacity of the measurement chamber 1 overflows from the outflow port 12. The inflow port 11 and the outflow port 12 constitute a test gas flow system for allowing the test gas to continuously flow into the measurement chamber 1.

Moisture (water vapor) contained in the test gas in the measurement chamber 1 is absorbed into the sample 100. Thus, the sample 100 is changed in weight in accordance with the amount of moisture absorbed into the sample 100. In the measurement chamber 1, a weight detector 3 for detecting a weight of the sample 100 is provided. The weight detector 3 detects a weight of the sample 100 placed on a mounting table 31, and the moisture amount of the sample 100 is measured based on a detection result of the weight thereof.

### 2. Composition of Sample

Fig. 2 is a schematic sectional view illustrating an example of the composition of a sample 100. In the present embodiment, the sample 100 to be measured is a multilayer film in which a plurality of layers 101, 102, and 103 is laminated, for example. More specifically, the sample 100 is a polarizing plate in which a base film 101, a polarizer 102, an adhesive layer 103, and the like are laminated, for example. Each of the layers 101, 102, and 103 has a thickness of from a few micrometers to a few tens micrometers, for example.

The sample 100 is disposed in the measurement chamber 1, so that moisture contained in the test gas in the measurement chamber 1 is absorbed into the base film 101, the polarizer 102, and the adhesive layer 103, as indicated by arrows in Fig. 2. Each of the layers 101, 102, and 103 has its own ease of moisture accumulation, and moisture contained in the test gas in the measurement chamber 1 accumulates little by little in the layers and interfaces between the corresponding layers. A ratio of moisture (moisture content) contained in each of the layers 101, 102, and 103 is changed in accordance with a water vapor concentration of the test gas in the measurement chamber 1.

The ease of moisture accumulation of each of the layers 101, 102, and 103 constituting the sample 100 is different between a single layer state and a laminated state of each of the layers 101, 102, and 103. It is considered that this is caused by receiving interaction such as internal pressure due to difference in linear expansion of each of the laminated layers 101, 102, and 103, and existence of internal stress caused through a stretching process. In the present embodiment, a main object is to measure the moisture amount of the sample 100 in a state where the layers 101, 102, and 103 are laminated, and to analyze characteristics of each of the layers 101, 102, and 103 based on the measurement result.

### 3. Configuration of Dynamic Moisture Absorption-Desorption Property Evaluation Apparatus

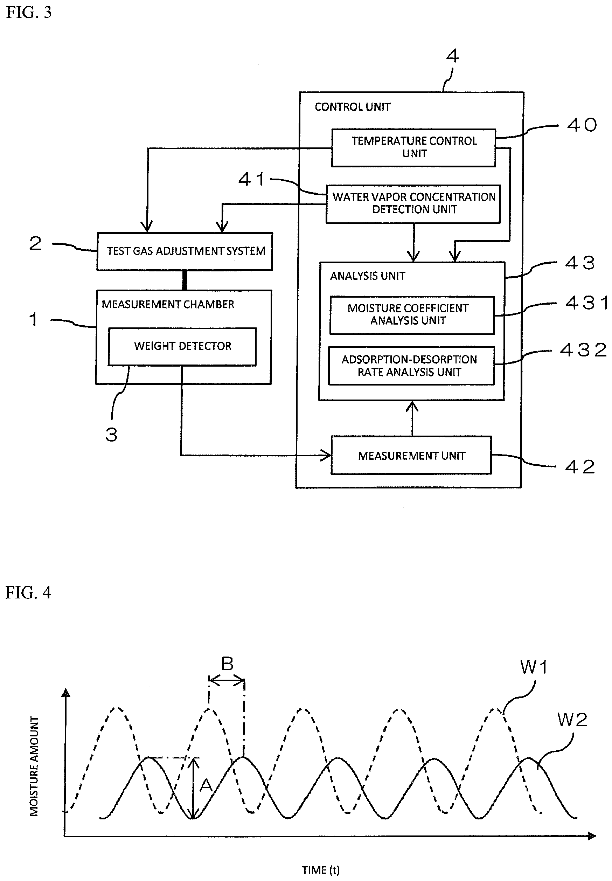

Fig. 3 is a block diagram illustrating an example of a configuration of a dynamic moisture absorption-desorption property evaluation apparatus. In addition to the configuration described above, the dynamic moisture absorption-desorption property evaluation apparatus of the present embodiment also includes a test gas adjustment system 2, a control unit 4, and the like, for example.

The test gas adjustment system 2 adjusts a water vapor concentration and temperature of a test gas flowing into the measurement chamber 1 from the inflow port 11. Controlling the test gas adjustment system 2 enables each of the water vapor concentration and the temperature of the test gas in the measurement chamber 1 to be adjusted to any value. That is, the water vapor concentration and the temperature of the test gas in the measurement chamber 1 can be kept constant or varied.

The control unit 4 is composed of a control device such as a computer. The control unit 4 includes a central processing unit (CPU), for example, and serves as a temperature control unit 40, a water vapor concentration control unit 41, a measurement unit 42, an analysis unit 43, and the like, when the CPU executes a program. The analysis unit 43 includes a moisture coefficient analysis unit 431 and an adsorption-desorption rate analysis unit 432.

The temperature control unit 40 causes the test gas adjustment system 2 to change temperature of the test gas in the measurement chamber 1. The water vapor concentration control unit 41 causes the test gas adjustment system 2 to change a water vapor concentration in the test gas in the measurement chamber 1. The measurement unit 42 measures the moisture amount of the sample 100 based on the weight of the sample 100 detected by the weight detector 3. That is, a difference between a weight of the sample 100 in a state where no moisture is contained and a weight of the sample 100 after being exposed to the test gas containing moisture in the measurement chamber 1 is calculated as the moisture amount of the sample 100.

The analysis unit 43 analyzes characteristics of the sample 100 based on a control mode of the test gas adjustment system 2 in each of the temperature control unit 40 and the water vapor concentration control unit 41, and a measurement result of the moisture amount of the sample in the measurement unit 42. In the present embodiment, a moisture coefficient of the sample 100 can be analyzed by the moisture coefficient analysis unit 431, and an adsorption-desorption rate can be analyzed by the adsorption-desorption rate analysis unit 432. Here, the moisture coefficient means a value representing ease of moisture accumulation. The adsorption-desorption rate is a value representing ease of adsorption and desorption of moisture.

### 4. Method for Analyzing Sample

Fig. 4 is a graph for describing a method for analyzing the sample 100. In the present embodiment, the water vapor concentration control unit 41 causes the test gas adjustment system 2 to periodically change a water vapor concentration in the test gas in the measurement chamber 1, and then the characteristics of the sample 100 are analyzed based on a measurement result of the moisture amount of the sample in the measurement unit 42 at that time. In Fig. 4, where the horizontal axis represents time and the vertical axis represents the moisture amount (water vapor concentration), the moisture amount (output) measured by the measurement unit 42 is indicated by the solid line, and periodic change (input) of the water vapor concentration in the test gas in the measurement chamber 1 is indicated by the broken line in association with the moisture amount.

In the example of Fig. 4, the water vapor concentration control unit 41 controls the test gas adjustment system 2 such that the water vapor concentration in the test gas in the measurement chamber 1 varies as a sine curve. However, variations of the water vapor concentration in the test gas in the measurement chamber 1 may be a periodic change, and thus the water vapor concentration can be varied in other various modes such as a cosine curve. In this case, a waveform W1 indicating the periodic change of the water vapor concentration in the test gas in the measurement chamber 1, being input, is shifted from a waveform W2 indicating the periodic change of the moisture amount of the sample measured by the measurement unit 42, being output. The present invention is configured to widely analyze the characteristics of the sample 100 by analyzing (waveform analysis) a shift of output from that of input, such as described above.

Specifically, a height A of the waveform W2 of the moisture amount of the sample, which corresponds to the waveform W1 of the change of the water vapor concentration in the test gas, is a value corresponding to ease of moisture accumulation (moisture coefficient) of the sample 100, and a shift B (shift in time) of the waveform W2 from the waveform W1 is a value corresponding to an adsorption-desorption rate of the sample 100. Thus, when these values A and B each are considered as a complex number, the characteristics of the sample 100 can be analyzed by using a complex number (A + B × i) where a moisture coefficient corresponding to A is a real part (Re), and an adsorption-desorption rate corresponding to B is an imaginary part (Im).

In addition, when a frequency of the periodic change of the water vapor concentration in the test gas in the measurement chamber 1, being input, is changed stepwise or continuously, characteristics of any layer and interlayer also can be evaluated from frequency dependence of the water vapor concentration in the test gas. That is, even when the sample 100 in which the plurality of layers 101, 102, and 103 is laminated as illustrated in Fig. 2 is measured in a state where the layers are laminated, changing the frequency of the waveform W1 of input enables analysis of characteristics (moisture coefficient and adsorption-desorption rate) of any one of the layers and interfaces corresponding to the frequency. Specifically, at each frequency, there performed analysis (waveform analysis) of a shift between the waveform W1 indicating periodic change of the water vapor concentration in the test gas in the measurement chamber 1, and the waveform W2 indicating periodic change of the moisture amount of the sample measured by the measurement unit 42, with respect to the change of the water vapor concentration in the test gas. At this time, it is possible to evaluate an internal state of the sample from a trajectory acquired by plotting characteristics at each frequency on a Nyquist diagram or a Bode diagram.

The analysis method as described above can be performed at each temperature by changing temperature in the measurement chamber 1. That is, when the temperature control unit 40 changes temperature of the test gas in the measurement chamber 1, and the water vapor concentration control unit 41 changes the water vapor concentration in the test gas in the measurement chamber 1 at each temperature, a shift in change of the moisture amount of the sample measured by the measurement unit 42 from change of the water vapor concentration at each temperature may be calculated. For example, waveform analysis as described above may be performed by changing the water vapor concentration in the test gas in the measurement chamber 1 at different respective temperatures such as 40°C, 50°C, 60°C, and 70°C.

### 5. Function Effect

In the present embodiment, when the water vapor concentration in the test gas in the measurement chamber 1 is varied (control step), and a shift B in change of the moisture amount of the sample measured by the measurement unit 42 from the change of the water vapor concentration is calculated by the analysis unit 43 (analysis step), the characteristics of the sample 100 can be analyzed based on the shift B. The calculated shift B in the change of the moisture amount of the sample corresponds to the adsorption-desorption rate in the sample 100, as described above. Thus, it is possible to analyze not only the moisture amount of the sample 100 but also the adsorption-desorption rate of the sample, so that characteristics of the sample 100 can be widely analyzed.

In the present embodiment, the characteristics of the sample 100 can be widely analyzed by waveform analysis of comparing the waveform W1 indicating the periodic change of the water vapor concentration in the test gas in the measurement chamber 1, with the waveform W2 indicating the periodic change of the moisture amount of the sample measured by the measurement unit 42, with respect to the change of the water vapor concentration in the test gas. Specifically, as described above, the ease of moisture accumulation (moisture coefficient) can be analyzed based on the height A of the waveform W2, and the adsorption-desorption rate can be analyzed based on the shift B between the waveforms W1 and W2. The waveform W2 indicating the periodic change of the moisture amount of the sample shows the characteristics of the sample 100 at the position corresponding to a frequency of the waveform W1 indicating the periodic change of the water vapor concentration in the test gas, so that characteristics in any layer in the sample 100 can be evaluated by arbitrarily setting the frequency as described above.

### 6. Modification

In the above embodiment, the configuration in which the water vapor concentration control unit 41 periodically changes the water vapor concentration in the test gas in the measurement chamber 1 by controlling the test gas adjustment system 2 is described. However, the configuration may be such that the water vapor concentration in the test gas in the measurement chamber 1 may be changed regularly or irregularly in a mode other than the periodic change.

The sample 100 is not limited to a multilayer film, and may be a single film. In addition, the dynamic moisture absorption-desorption property evaluation apparatus according to the present invention can also measure the sample 100 other than a film.

While the configuration of the dynamic moisture absorption-desorption property evaluation apparatus is described in the above embodiment, it is also possible to provide a program (dynamic moisture absorption-desorption property evaluation program) for causing a computer to serve as the control unit 4 of the dynamic moisture absorption-desorption property evaluation apparatus. In this case, the program may be provided while being stored in a storage medium, or the program itself may be provided.

The present invention is also applicable to a dynamic absorption-desorption property evaluation apparatus, a method for evaluating a dynamic absorption-desorption property, and a dynamic absorption-desorption property evaluation program, for evaluating a dynamic absorption-desorption property not only for water vapor but also for various specimens such as oxygen and carbon dioxide.

### DESCRIPTION OF REFERENCE NUMERALS

- 1: measurement chamber
- 2: test gas adjustment system
- 3: weight detector
- 4: control unit
- 10: casing
- 11: inflow port
- 12: outflow port
- 31: mounting table
- 40: temperature control unit
- 41: water vapor concentration control unit
- 42: measurement unit
- 43: analysis unit
- 100: sample
- 101: base film
- 102: polarizer
- 103: adhesive layer
- 431: moisture coefficient analysis unit
- 432: adsorption-desorption rate analysis unit
- W1: waveform
- W2: waveform
- A: height of waveform W2
- B: shift of waveform W2 from waveform W1 (shift in time)

## Claims

1. A dynamic moisture absorption-desorption property evaluation apparatus comprising:
a measurement chamber in which a sample is disposed;
a test gas flow system that allows a test gas to continuously flow into the measurement chamber;
a test gas adjustment system that adjusts a water vapor concentration of the test gas in the measurement chamber;
a measurement unit that measures a moisture amount of the sample in the measurement chamber;
a control unit that causes the test gas adjustment system to change the water vapor concentration in the test gas; and
an analysis unit that analyzes a shift in change of the moisture amount of the sample measured by the measurement unit from change of the water vapor concentration in the test gas, when the water vapor concentration in the test gas is changed by the control unit.

2. The dynamic moisture absorption-desorption property evaluation apparatus according to claim 1, wherein
the control unit causes the test gas adjustment system to periodically change the water vapor concentration in the test gas, and
the analysis unit performs waveform analysis based on a waveform indicating a periodic change of the water vapor concentration in the test gas, and a waveform indicating a periodic change of the moisture amount of the sample measured by the measurement unit, with respect to the change of the water vapor concentration in the test gas.

3. The dynamic moisture absorption-desorption property evaluation apparatus according to claim 2, wherein
the control unit changes a frequency of periodic change of the water vapor concentration in the test gas stepwise or continuously, and
the analysis unit performs waveform analysis at each frequency.

4. The dynamic moisture absorption-desorption property evaluation apparatus according to any one of claims 1 to 3, wherein
the test gas adjustment system adjusts the water vapor concentration and temperature in the test gas,
the control unit causes the test gas adjustment system to change the water vapor concentration and temperature in the test gas, and
the analysis unit calculates a shift in change of the moisture amount of the sample measured by the measurement unit from change of the water vapor concentration in the test gas at each temperature, when temperature of the test gas is changed by the control unit and the water vapor concentration in the test gas at each temperature is changed by the control unit.

5. A dynamic absorption-desorption property evaluation apparatus comprising:
a measurement chamber in which a sample is disposed;
a test gas flow system that allows a test gas to continuously flow into the measurement chamber;
a test gas adjustment system that adjusts a concentration of a specimen in the test gas in the measurement chamber;
a measurement unit that measures an absorption-desorption amount of a specimen in the sample in the measurement chamber;
a control unit that causes the test gas adjustment system to change the concentration of the specimen in the test gas; and
an analysis unit that analyzes a shift in change of the absorption-desorption amount of the specimen in the sample measured by the measurement unit from change of the concentration of the specimen in the test gas, when the concentration of the specimen in the test gas is changed by the control unit.

6. A method for evaluating a dynamic moisture absorption-desorption property, the method comprising the steps of:
disposing a sample in a measurement chamber;
allowing a test gas to continuously flow into the measurement chamber;
performing control for changing a water vapor concentration in the test gas; and
performing analysis of analyzing a shift in change of a moisture amount of the sample from change of the water vapor concentration in the test gas, when the water vapor concentration in the test gas is changed in the step of performing control.

7. The method for evaluating a dynamic moisture absorption-desorption property according to claim 6, wherein
the water vapor concentration in the test gas is periodically changed in the step of performing control, and
waveform analysis is performed in the step of performing analysis based on a waveform indicating periodic change of the water vapor concentration in the test gas and a waveform indicating periodic change in the moisture amount of the sample, with respect to the change of the water vapor concentration in the test gas.

8. The method for evaluating a dynamic moisture absorption-desorption property according to claim 7, wherein
a frequency of the periodic change of the water vapor concentration in the test gas is changed stepwise or continuously in the step of performing control, and
the waveform analysis is performed at each frequency in the step of performing analysis.

9. The method for evaluating a dynamic moisture absorption-desorption property according to any one of claims 6 to 8, wherein
the water vapor concentration and temperature in the test gas are changed in the step of performing control, and
a shift in change of the moisture amount of the sample from change of the water vapor concentration in the test gas at each temperature is calculated, when the temperature of the test gas is changed and the water vapor concentration in the test gas is changed at each temperature, in the step of performing analysis.

10. A method for evaluating a dynamic absorption-desorption property, the method comprising the steps of:
disposing a sample in a measurement chamber;
allowing a test gas to continuously flow into the measurement chamber;
performing control for changing a concentration of a specimen in the test gas; and
performing analysis of analyzing a shift in change of an absorption-desorption amount of a specimen in the sample from change of the concentration of the specimen in the test gas, when the concentration of the specimen in the test gas is changed in the step of performing control.

11. A dynamic moisture absorption-desorption property evaluation program configured to evaluate a dynamic moisture absorption-desorption property of a sample in a measurement chamber, the program causing a computer to execute the steps of:
allowing a test gas to continuously flow into the measurement chamber;
performing control for changing a water vapor concentration in the test gas; and
performing analysis of analyzing a shift in change of a moisture amount of the sample from change of the water vapor concentration in the test gas, when the water vapor concentration in the test gas is changed in the step of performing control.

12. The dynamic moisture absorption-desorption property evaluation program according to claim 11, wherein
the water vapor concentration in the test gas is periodically changed in the step of performing control, and
waveform analysis is performed in the step of performing analysis based on a waveform indicating periodic change of the water vapor concentration in the test gas and a waveform indicating periodic change of the moisture amount of the sample, with respect to the change in the water vapor concentration in the test gas.

13. The dynamic moisture absorption-desorption property evaluation program according to claim 12, wherein
a frequency of the periodic change of the water vapor concentration in the test gas is changed stepwise or continuously in the step of performing control, and
the waveform analysis is performed at each frequency in the step of performing analysis.

14. The dynamic moisture absorption-desorption property evaluation program according to any one of claims 11 to 13, wherein
the water vapor concentration and temperature in the test gas are changed in the step of performing control, and
a shift in change of the moisture amount of the sample from change of the water vapor concentration in the test gas at each temperature is calculated, when the temperature of the test gas is changed and the water vapor concentration in the test gas is changed at each temperature, in the step of performing analysis.

15. A dynamic absorption-desorption property evaluation program configured to evaluate an absorption-desorption property of a sample in a measurement chamber, the program causing a computer to execute the steps of:
allowing a test gas to continuously flow into the measurement chamber;
performing control for changing a concentration of a specimen in the test gas; and
performing analysis of analyzing a shift in change of an absorption-desorption amount of a specimen in the sample from change of the concentration of the specimen in the test gas, when the concentration of the specimen in the test gas is changed in the step of performing control.
